(19) Europäisches Patentamt
European Patent Office
Office européen des brevets

(11) EP 1 776 116 B1

(12) **EUROPEAN PATENT SPECIFICATION**

(45) Date of publication and mention of the grant of the patent:
**29.09.2010 Bulletin 2010/39**

(21) Application number: **05769652.8**

(22) Date of filing: **27.07.2005**

(51) Int Cl.:
***A61K 31/44*** *(2006.01)* ***A61K 31/4178*** *(2006.01)*
***A61K 31/4192*** *(2006.01)* ***A01N 43/647*** *(2006.01)*
***A01N 43/56*** *(2006.01)* ***A01N 43/74*** *(2006.01)*
***A01N 43/78*** *(2006.01)* ***A01N 43/40*** *(2006.01)*
***A01N 43/08*** *(2006.01)* ***A61P 33/00*** *(2006.01)*

(86) International application number:
**PCT/EP2005/053667**

(87) International publication number:
**WO 2006/010767 (02.02.2006 Gazette 2006/05)**

(54) **VETERINARY COMPOSITION COMPRISING AN ARYLPYRAZOLE AND A NITROENAMINE WITH ENHANCED ANTIPARASITIC ACTIVITY**

VETERINÄRMEDIZINISCHE ZUSAMMENSETZUNG MIT EINEM ARYLPYRAZOL UND EINEM NITROENAMIN MIT ERHÖHTER ANTIPARASITISCHER AKTIVITÄT

COMPOSITION VETERINAIRE COMPRENANT UN ARYLPYRAZOLE ET UNE NITRO-ENAMINE QUI PRESENTE UNE MEILLEURE ACTIVITE ANTIPARASITAIRE

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.07.2004 EP 04103616**

(43) Date of publication of application:
**25.04.2007 Bulletin 2007/17**

(73) Proprietor: **Intervet International BV**
**5831 AN Boxmeer (NL)**

(72) Inventors:
- **MERTENS, Christina**
**5831 AN Boxmeer (NL)**
- **DOHRMANN, Heike**
**55270 Sörgenloch (DE)**

(74) Representative: **Stumm, Karin**
**Intervet International BV**
**Patent Department**
**Wim de Körverstraat 35**
**P.O. Box 31**
**5830 AA Boxmeer (NL)**

(56) References cited:
**EP-A- 0 302 389** **EP-A- 0 412 849**
**US-A1- 2002 058 683**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 776 116 B1

## Description

The present invention relates to veterinary compositions for the control of parasitic insects and acarids and their use for the manufacture of a veterinary medicament for the control of parasitic insect- and acarid - infestations on animals.

A number of pests and parasites can infest or infect domestic animals such as cattle, horses, pigs, sheep and also companion animals such as cats and dogs. These pests and parasites are of great nuisance to both the animals and their owners. External parasites such as ticks, mites, lice and fleas irritate the animals and can cause disease, either by themselves, or by carrying vector transmitted pathogens.

Ticks are important blood feeding arthropod parasites that belong together with mites to the order *Acarina.* Ticks produce injury after infestation of animals in three respects: direct damage caused by parasitism such as local injury and blood loss; by toxins injected by the parasites and by the transmission of diseases. Especially in companion animals, ticks may be the source of tick transmitted diseases.

Fleas (*Ctenocephalides felis* and *Ctenocephalides canis*) are the most common ectoparasites of cats and dogs. Flea infestation of dogs and cats has unpleasant consequences not only for the animal to be treated but also for the owner. Flea infestation leads, for example, to local irritation or troublesome itching and often results in intense scratching. A large number of animals become allergic to the saliva of the fleas causing itchy local reactions at the sites of flea bites often leading to lesions and secondary infection due to scratching. Furthermore, flea- and tick- infested animals are constantly exposed to the risk of infestation by parasite transmitted pathogens such as *Rickettsia, Protozoa* or *Taenia* e.g. *Dipyllidium,* a species of tape worm which is transmitted by fleas.

Safe, effective ways to eliminate these parasites are desired for the companion animal's well-being, for the well-being and comfort of its human associate and for the prevention of losses in livestock. Thus, there continues to be a need for compounds and combinations thereof which can be used as active agents against parasites, especially those such as fleas and ticks that afflict companion animals, and which are effective at low application rates, are selective in biological action and have low toxicity.

In EP 0412849 certain aryl-1,2,3-triazoles and arylpyrazoles are disclosed in which an imidazol(in)e group is attached directly or indirectly at its 2-position to the triazole or pyrazole ring and which have pesticidal activity. For certain of the compounds disclosed in EP 0412849 systemic activity against ectoparasitic insects after oral application to an animal is described But no combination with a nitroenamine compound is suggested.

Certain nitroenamines are described in EP 0302389 as contact insecticides and contact acaricides. EP 0616494 discloses that compounds out of this group that are 1-[N-(halo-3-pyridylmethyl)]-N-methylamino-l-alkylamino-2-nitroethylene derivatives show a pronounced activity against fleas. US 2002/58683 describes a combination of lufenuron and nitenpyram. From EP 0616494 it is known that such compounds may also be administered orally to host animals but no combination with a arylpyrazole compound is suggested..

A compound out of the group of nitroenamine compounds of formula (II) is commercially available for the control of acute flea infestation in companion animals comprising Nitenpyram (Capstar®, Novartis Inc.).

It is known that, nitroenamines like Nitenpyram are rapidly absorbed via the gastro-intestinal tract and distributed in the blood when they are administered orally, e.g. as tablets, but are excreted rapidly via the urine. Therefore the persistence of antiparasitic activity after single administration is rather short.

Attempts to prolong the persistence of antiparasitic activity of such compounds e.g. by increasing the dose were not successful. For Nitenpyram a half-life in the blood of 7.7 hours for cats and 2.8 hours for dogs and a mean residence time of 10.2 hours for cats and 4.1 hours for dogs have been reported (Dryden MW, et al: Proceedings of the Annual Meeting of the American Association of Veterinary Parasitology 44: pp 1-9, 1999). This is much too short to achieve a significant improvement in persistent efficacy by means of increasing the dose. A very steep rise in blood values after application is observed as is an equally rapid flattening of the curve, without a significant influence on prolonged bioavailability. Recently it was reported that after 72 hours levels of nitenpyram-in host blood were not longer lethal to fleas (Rust MK et al: J. Med. Entomol. 40(5): pp 678 - 681, 2003).

This limited persistence of antiparasitic activity is a major obstacle for the use of these compounds as single active ingredient in flea control regimens.

Because of the limited persistence of antiparasitic activity tablets or injections have to be administered at short intervals, preferably every other day, which means the owner has to repeat the treatment or visit the veterinarian too frequently. An intensive treatment concept of this kind requires a great amount of compliance. Experience has shown that this causes stress to the animal and the owner after only a short time. This often manifests itself as an aversion to the treatment and leads to discontinuation of the treatment. Therefore, prolonging the systemic action against fleas of the nitroenamine compounds would be desirable.

Based on the information from the prior art it was expected that a fast onset of efficacy against fleas but a very limited persistence of activity would be observed.

Surprisingly it has been found that a composition comprising a combination of an arylpyrazole compound of

formula (I) and an nitroenamine compound of formula (II) show a persistent activity against fleas for an extended period of time in a dosage that is used to control ticks. This enhanced kill activity against acarids and fleas provides excellent control of the most important ectoparasites in companion animals.

This is rather unexpected because it is known that nitroenamine compounds alone have a short persistence of activity against fleas as outlined above. The arylpyrazole compound alone generally has limited activity against fleas in a dosage used to control ticks.

The present invention therefore provides a veterinary composition for the control of parasitic insects and acarids comprising a combination of an arylpyrazole compound of formula (I)

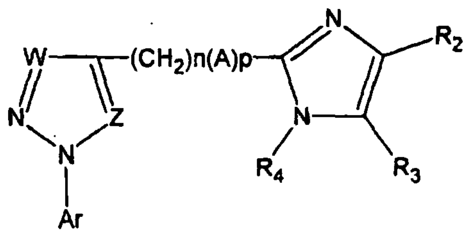

(I)

in which

Ar is 2,6-dichloro-4-trifluoromethylphenyl or 2-nitro-4-trifluoromethylphenyl;

A is $S(O)_m$, -CH=CH-, O or NH;

W is N and Z is $CR^5$; or W is $CR^1$ and Z is N or $CR^5$;

$R^1$ is hydrogen, optionally substituted alkyl, halogen or $R^{20}S(O)_q$;

$R^2$ and $R^3$ are hydrogen, alkyl, alkenyl or alkynyl, each of which is optionally substituted, aryl, cyano, halogen, nitro, $YR^{20}$, $S(O)_2NR^8R^9$, CHO, $NR^8R^9$ or $CYNR^8R^9$;

$R^4$ is hydrogen, optionally substituted alkyl, optionally substituted alkenyl, acyl or optionally substituted alkoxycarbonyl;

$R^5$ is hydrogen, alkyl, optionally substituted amino or halogen;

$R^8$ and $R^9$ are the same or different and are hydrogen, optionally substituted alkyl, acyl or aryl;

$R^{20}$ is optionally substituted alkyl;

Y is O or S;

m is 0, 1 or 2;

p is 0 or 1;

n is 0, 1 or 2; and

q is 0, 1 or 2,

and in which a) any alkyl, alkoxy and alkylthio groups are of 1 to 4 carbon atoms; b) any alkenyl or alkynyl groups are of 2 to 5 carbon atoms; c) any substituted alkyl, alkoxy, alkylthio, alkenyl or alkynyl group is substituted by one or more of the same or different groups selected from halogen, YR , dihalocyclopropyl, cyano, nitro, optionally substituted amino, acyloxy and aryl: d) any aryl group is phenyl, optionally substituted, by halogen, alkyl, haloalkyl, alkoxy, haloalkoxy, alkylthio, haloalkylthio, haloalkylsulphonyl, cyano or nitro; e) any acyl group is alkanoyl of 1 to 4 carbon atoms, or alkylsulphonyl or haloalkylsulphonyl; and f) any optionally substituted amino groups are of formula $NR^8R^9$, with the proviso that when W is $CR^1$ and Z is $CR^5$ and n and p are both 0, $R^4$ is not alkyl;

and a nitroenamine compound of formula (II)

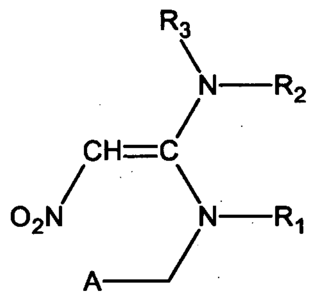

(II)

wherein

R1 is hydrogen, $C_1$-$C_6$ alkyl or $C_3$-$C_7$ cycloalkyl;
R2 is hydrogen, $C_1$-$C_6$ alkyl or $C_3$-$C_7$ cycloalkyl;
R3 is hydrogen or $C_1$-$C_6$ alkyl;
A is heterocyclyl which is unsubstituted or substituted once or repeatedly by identical or different halogen atoms;
and a physiologically acceptable formulation excipient.
Arylpyrazole compounds of formula (I) have been described in EP 0412849. Methods for the preparation of these compounds are also disclosed in EP 0412849.
Nitroenamine compounds falling within the scope of formula (II) including processes for their preparation are described in EP 0302389.

The alkyl groups present in the definitions of the substituents may be straight -chain or branched, depending on the number of carbon atoms, and they may be for example methyl, ethyl, propyl, butyl, pentyl or hexyl, as well as the branched isomers thereof, for example isopropyl, isobutyl, sec.-butyl, tert-butyl, isopentyl, neopentyl or isohexyl. $C_3$ to $C_7$ cycloalkyl is cyclopropyl, cyclobutyl, cyclopentyl, cyclohexyl or cycloheptyl.
Halogen atoms are e.g. fluorine, chlorine, bromine or iodine, preferably fluorine or chlorine, especially chlorine, whereby a partially or completely halogenated substituent may contain one or more identical or different halogen atoms.
In the context of the present invention, heterocyclyl is understood to mean aliphatic or aromatic cyclic radicals, which contain at least one oxygen, sulphur or nitrogen atom. Five- and six -membered heterocycles are preferred. Heterocyclyl typically includes groups such as dioxolanyl, pyrrolidonyl, piperidinyl, morpholinyl, pyridyl, pyrrolyl, pyrryl, furyl, thienyl, imidazolyl, tetrahydrofuryl, tetrahydropyranyl, dihydrofuryl, dihydropyranyl, isoxazoyl,oxazolyl, thiazolyl, oxazolinyl, oxazolidinyl, and imidazolinyl.
Preference is given especially to those which are un-substituted or have one or two halogen atoms. Halogen in this case denotes fluorine, chlorine or bromine, but especially chlorine. Of these heterocyclic radicals, pyridyl, thiazolyl, and tetrahydrofuryl are especially notable.
Especially preferred are 5,6-dichloropyridin-3-yl, especially 6-chloropyridin-3-yl, but also 5-chlorothiazol-3-yl and tetrahydrofur-3-yl, in particular 6-chloropyridin-3-yl.
A prominent representative of the nitroenamine compounds of formula (II) is Nitenpyram (INN) of formula (III).

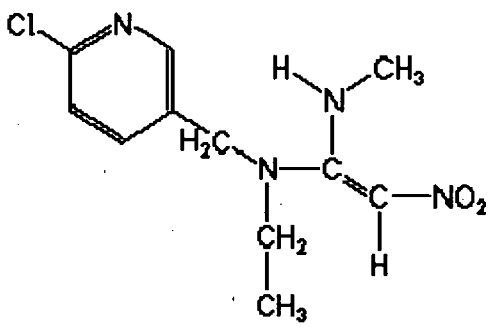

(III)

IUAPC name (E)-N-(6-chloro-pyridin-3 ylmethyl)-N-ethyl-N-methyl-2-methyl-2-nitrovinylidenediamine. Nitenpyram and its preparation are described in EP 0302389 as Example 41 on page 63.

A prominent representative of an arylpyrazole compound of formula (I) for use in the composition according to the invention is 5-chloro-1-(2,6-dichloro-4-trifluoromethylphenyl)-4-(4,5-dicyano-1H-imidazol-2-yl)-3-isopropyl-1H-pyrazole of formula (IV):

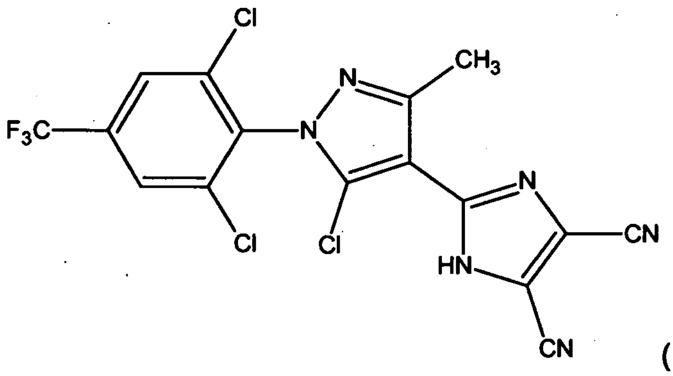

(IV)

This compound is indicated as compound 22c in EP 0412849. The synthesis of this compound is disclosed in Example 22b of EP 0412849. In the remainder of this application this compound will be referred to as "compound 22c".
In another embodiment the invention relates to the use of the composition according to the invention for the manufacture of a veterinary medicament for the control of parasitic insect- and acarid- infestations on animals.
In order to form the composition according to the invention the active ingredients may be present in the dosage form as true mixtures, but they may also be administered individually in separate dosage forms and form mixtures only when they are in the host organism.
In case compounds of formula (I) and the compound of formula (II) are administered individually in separate dosage forms they are administered in parallel. Parallel means that the active ingredients may be administered at the same time, that is simultaneously, but they may also be administered sequentially, that is one after the other i.e. so that they are present together for certain periods at the latest in the host organism, so that the desired effect arises.
The active ingredients are preferably administered simultaneously. When given simultaneously the composition according to the invention is preferably presented as a single dosage form comprising both the compound of formula (I) and of the formula (II) in a single formulation.
Preferably the combination of active ingredients according to the invention is administered systemically. Systemic administration is the administration of the combination at a site remote from the site where the parasite resides so that the active compound can be ingested by the feeding parasite along with the blood, body fluids or tissue such as skin of the host animal, and can then exhibit activity against the parasite.
In accordance with the present invention a systemic administration is achieved by several forms. For example, the composition may be administered in an oral formulation or parenterally, e.g. by injection, as implant or as a bolus or by a topical administration method like pour-on or spot-on.
The compositions of the invention are preferably administered in an oral formulation. The term "oral formulation" means that the active ingredients are formulated into a product suitable for administering to the animal via the mouth. These formulations include, but are not limited to tablets, capsules, liquids, gels, pastes, oral sprays, buccal formulations, powders, granules, chewable treats or animal feeds containing the active ingredients.
The composition does not necessarily have to be administered to the animal directly. Oral administration includes, for example, the administration of animal food, e.g. for dogs or cats which contains the composition according to the invention already with therein. The composition can be administered e.g. as biscuits or as titbits, as chews, as capsules or tablets, in a form that can be dripped onto the food or into the drinking water, or in other forms that can be mixed with the animal food.
Other forms of non-direct oral administration include for example the application of the composition onto the coat of the animal and its later ingestion during the self cleaning of the animal.
Preferably the compositions according to the invention are provided as tablets, preferably chewable tablets that can be given to, for example dogs or cats, as a treat.
Conventional tablets generally comprise the active ingredients, a diluent to assist in increasing the powder mass to a convenient size and improve compressibility, a binder to hold the compressed powder together' and a lubricant to assist in densification and ejection from the tablet die. They may also contain a disintegrate to improve disintegration and dissolution, as well as stabilizers, colours and flavours. Tablets are often coated to improve appearance or taste or to alter the dissolution properties. Tablets can be designed to dissolve fast or slowly, and depending on the actual volume and compressibility of the drug, they may be large or small. They can be made chewable or to dissolve under the tongue

or in the pouch of the cheek. They may contain further additives which stimulate voluntary ingestion by the animal such as suitable odorous substance, taste substances, scents or flavourings.

The composition according to the invention can be also administered as liquid formulation. Conventional liquid formulations for oral administration are usually solutions, suspensions or emulsions of the active ingredients together with suitable diluents, solvents, flavours and colours to form a dosage form.

The compositions according to the current invention conventionally further comprise physiologically acceptable formulation excipients known in the art e.g. as described in "Gennaro, Remington: The Science and Practice of Pharmacy" (20th Edition, 2000) incorporated by reference herein. All such components, carriers and excipients must be substantially pharmaceutically or veterinary pure and non-toxic in the amounts employed and must be compatible with the active ingredients.

The compositions of the invention are intended for use for controlling a parasitic insect- and acarid infestation. The term "controlling a parasitic insect- and acarid infestation" refers to preventing, reducing or eliminating an infestation by such parasites on animals preferably by killing the insects and/ or acarids within hours or days.

The term "parasitic insect- and acarid" refers to ectoparasites e.g. insect and acarine pests that commonly infest or infect animals. Examples of such ectoparasites include the egg, larval, pupal, nymphal and adult stages of lice, fleas, mosquitoes, mites, ticks biting or nuisance fly species. Especially important are the adult stages of fleas and ticks.

In general, the composition according to the invention will contain an effective amount of the active ingredients, meaning a non-toxic but sufficient amount to provide the desired control effect. A person skilled in the art using routine experimentation may determine an appropriate "effective" amount in any individual case. Such an amount will depend on the age, condition, weight and type of the target animal.

The tablets may be formulated to contain an amount of active ingredients that is adjusted to animals in a specific weight range.

The animals may receive a monthly, weekly or daily dosage, optionally preceded by a higher loading dose (initial higher dose). The treatment can, for example, be continuing or seasonal.

Preferably the treatment is carried out so as to administer to the animal a composition comprising a dose from 0.1 to 20 mg/ kg bodyweight and in particular from 0.5 to 10 mg/ kg bodyweight and most preferably from 1 to 5 mg/ kg bodyweight of the compound of formula (I) and a dose from 0.1 to 50 mg/ kg bodyweight and in particular from 1 to 30 mg/ kg bodyweight and most preferably from 5 to 15 mg/ kg bodyweight of compound of formula (II) to be administered at weekly interval. These dosages have been proven to be effective, especially in companion animals such as dogs or cats.

Another aspect of the invention is a kit useful in the treatment of a parasite infestation of insects and/or acarids in an animal, which comprises a compound of formula (I) and of formula (II) in a veterinary acceptable formulation and instructions for the control of parasitic insect- and acarid- infestations on animals.

The compositions of the present invention may be prepared in a manner known per se for example by means of conventional mixing, granulating, coating, dissolving or lyophilising processes.

In general the composition according to the current invention can be administered to all species of animals that have insect- or acarid- pest infestation. The recipient of the formulation may be a livestock animal, e.g. sheep, cattle, pig, goat or poultry; a laboratory test animal, e.g. guinea pig, rat or mouse; or a companion animal, e.g. dog, cat, rabbit, ferret or horse. The compositions according to the invention are especially suitable for use in companion animals, e.g. dogs, cats or ferrets.

**Example**

**Efficacy of different dosages of Nitenpyram in combination with compound 22c**

**Material and Methods:**

For all treatment groups, Compound 22c was administered orally at an initial dose of 4 mg/kg body weight'(bw) followed by a maintenance dose of 2 mg/kg bw in the second treatment week. The initial dose of 4 mg/kg Compound 22c was split into 2 x 2 mg/kg bw of Compound 22c applied to dogs on two consecutive days simultaneously with Nitenpyram.

For Nitenpyram a dosing schedule of 15 mg/kg bw (Group A), 10 mg/kg bw (Group B) and 5 mg/kg bw (Group C) was used. Group D remained untreated as a control.

Four groups of 3 dogs each were infested with fleas (*Ctenocephalides felis*) and ticks (*Rhipicephalus sanguineus*) before and repeatedly at different time points after treatment.

The parasite burden of individual dogs was assessed 48 hours after the first treatment and after each following infestation by removal and counting of ticks and fleas. Ticks and fleas were classified according to vitality (dead / alive).

The details of the study procedures are indicated below:

**Study animals**

| | |
|---|---|
| Species: | Domestic dog |
| Number: | 16 |
| Breed: | Beagle |
| Body Weight: | 11-17 kg |
| Age: | 3-6 years |
| Gender: | male and 1x male, gelded |

**Parasite infestation**

Dogs were experimentally infested with laboratory strains of *Rhipicephalus sanguineus* (80 unfed adults; sex ratio 1:1) and *Ctenocephalides felis* (100 adults, similar age; sex ratio approx. 1:1). Fleas and ticks were directly applied onto the back of the animal. During the time of parasite distribution over the host, the dogs were sedated.

Ticks (*Rhipicephalus sanguineus*) and fleas (*Ctenocephalides felis*) were infested on day -2, day +3, day +5, day +7, day +10 and day +12.

**Product specification**

<u>Compound 22c Tablet</u>

| | | |
|---|---|---|
| Active Ingredient: | 10 mg Compound 22c / tablet | |
| Dosage (Per os): | 4 mg/kg bw initial dose | (Group A, B, C) |
| | 2 mg/kg bw maintenance dose | (Group A, B, C) |

Excipients: Lactose Monohydrate, Corn starch, Pre-gelatinized starch, Silica, colloidal anhydrous, Sodium carboxymethyl-cellulose, Magnesium stearate

<u>Nitenpyram Tablet</u>

commercial product (Per os): Capstar® (Novartis)

| | | |
|---|---|---|
| Dosage: | 15 mg/kg bw | (Group A) |
| | 10 mg/kg bw | (Group B) |
| | 5 mg/kg bw | (Group C) |

**Treatment**

**Table 1: Infestation, treatment and assessment scheme**

| Day | Group | Number of dogs per group | Investigational veterinary product | Dose | Application |
|---|---|---|---|---|---|
| 0 | A<br>B<br>C | 3 | Compound 22c | 2 mg/kg BW | Per os |
| +1 | A<br>B | 3 | Compound 22c | 2 mg/kg BW | Per os |

(continued)

| Day | Group | Number of dogs per group | Investigational veterinary product | Dose | Application |
|---|---|---|---|---|---|
| | C | | | | |
| +7 | A<br>B<br>C | 3 | Compound 22c | 2 mg/kg BW | Per os |
| | | | | | |
| 0 | A<br>B<br>C | 3 | Nitenpyram | 15 mg/kg BW<br>10 mg/kg BW<br>5 mg/kg BW | Per os |
| +1 | A<br>B<br>C | 3 | Nitenpyram | 15 mg/kg BW<br>10 mg/kg BW<br>5 mg/kg BW | Per os |
| 7 | A<br>B<br>C | 3 | Nitenpyram | 15 mg/kg BW<br>10 mg/kg BW<br>5 mg/kg BW | Per os |

**Evaluation of Tick and Flea Numbers**

Parasite assessments on the dogs were made 48 hours after the first treatment and after each re-infestation on day +2, day +5, day +7, day +9, day +12 and day +14.

The flea count on each dog was conducted by combing until no flea was recovered for at least 5 minutes and the number of live fleas was recorded.

Ticks on the dogs were counted by palpation, removed where attached and classified.

**Calculation of Efficacy**

Efficacy calculation was based on the arithmetic means of the number of ticks / fleas on treated dogs compared to that of the control group. For calculation of efficacy (%), the following formula (according to Abbott's formula) is used:

$$\text{Efficacy} = 100 \times (mc - mr) / mc$$

Control group (mc): mean number of live fleas on the host animals
mean number of live ticks on the host animals
Treatment group (mr): mean number of live fleas on the host animals
mean number of live ticks on the host animals

## RESULTS

### EFFICACY AGAINST RHIPICEPHALUS SANGUINEUS

The numbers of ticks and percentage efficacy are given in Table 2. The percentage efficacy against ticks is given in Figure 1.

**Table 2: EFFICACY AGAINST RHIPICEPHALUS SANGUINEUS**

| Day after treatment | Group A Mean number of ticks | Group A Efficacy in percent | Group B Mean number of ticks | Group B Efficacy in percent | Group C Mean number of ticks | Group C Efficacy in percent |
|---|---|---|---|---|---|---|
| D +2 | 1.33 | 96.7 % | 3.33 | 91.7% | 1.67 | 95.9% |

(continued)

| Day after treatment | Group A Mean number of ticks | Group A Efficacy in percent | Group B Mean number of ticks | Group B Efficacy in percent | Group C Mean number of ticks | Group C Efficacy in percent |
|---|---|---|---|---|---|---|
| D +5 | 2.67 | 93.7 % | 3.00 | 92.9% | 2.33 | 94.5% |
| D +7 | 3.33 | 91.2 % | 0.33 | 99.1 % | 2.67 | 93.0 % |
| D +9 | 1.67 | 96.1 % | 0.33 | 99.2 % | 0.33 | 99.2 % |
| D +12 | 1.67 | 96.3 % | 0.67 | 98.5% | 0.00 | 100 % |
| D +14 | 1.67 | 93.8 % | 0.33 | 98.8% | 0.33 | 98.8% |

For group A treated with an initial dose of 4 mg/kg bw Compound 22c (split into 2 x 2 mg/kg bw on two consecutive days) plus 15 mg/kg bw Nitenpyram, an immediate tick efficacy of 96.7 % was achieved. Activity decreased slightly to 93.7 % and 91.2 % five and seven days after the first treatment. After re-treatment with 2 mg/kg Compound 22c plus 15 mg/kg bw Nitenpyram, in the 2$^{nd}$ week tick efficacy of 93.8 to 96.3 % was demonstrated.

Group B was treated with an initial dose of 4 mg/kg bw Compound 22c (split into 2 x 2 mg/kg bw on two consecutive days) plus 10 mg/kg bw Nitenpyram and showed an initial efficacy of 91.7 % that increased to 92.9 % and 99.1 % five and seven days after the first treatment. After re-treatment with 2 mg/kg bw Compound 22c plus 10 mg/kg bw Nitenpyram efficacy in the second week was in the range of 98.5 to 99.2 %.

For group C treated with an initial dose of 4 mg/kg bw Compound 22c (split into 2 x 2 mg/kg bw on two consecutive days) plus 5 mg/kg bw Nitenpyram, an immediate tick efficacy of 95.9 % was achieved. Five and seven days after first treatment 94.5 % and 93.0 % efficacy were demonstrated. Re-treatment with 2 mg/kg bw Compound 22c plus 5 mg/kg bw Nitenpyram increased efficacy to 98.8 up to 100 % in the second week.

Overall, excellent tick efficacy was demonstrated with a combination of Compound 22c and Nitenpyram. All treatment groups fulfilled the guideline requirements of at least 90 % tick efficacy ("Testing and evaluation of the efficacy of antiparasitic substances for the treatment and prevention of tick and flea infestation in dogs and cats" EMEA/CVMP/005/00).

**EFFICACY AGAINST CTENOCEPHALIDES FELIS**

The numbers of fleas and the percentage efficacy against fleas are given in Table 3. The percentage efficacy against fleas is given in Figure 2.

**Table 3: EFFICACY AGAINST CTENOCEPHALIDES FELIS**

| Day after treatment | Group A Mean number of fleas | Group A Efficacy in percent | Group B Mean number of fleas | Group B Efficacy in percent | Group C Mean number of fleas | Group C Efficacy in percent |
|---|---|---|---|---|---|---|
| D +2 | 0.0 | 100 % | 0.0 | 100.0 | 0.0 | 100.0 |
| D +5 | 0.0 | 100 | 0.0 | 100.0 | 0.0 | 100.0 |
| D +7 | 0.0 | 100 | 0.0 | 100.0 | 5.0 | 94.4 |
| D +9 | 0.0 | 100 | 0.0 | 100.0 | 0.0 | 100.0 |
| D +12 | 0.0 | 100 | 0.0 | 100.0 | 0.0 | 100.0 |
| D+14 | 0.0 | 100 | 0.0 | 100.0 | 7.0 | 92.1 |

Group A (15 mg/kg bw Nitenpyram) and group B (10 mg/kg bw Nitenpyram) achieved a flea efficacy of 100 % for the entire study period.

For group C (5 mg/kg bw Nitenpyram) a therapeutic and prophylactic flea efficacy of 100 % was achieved in the first week of study. Seven days after first treatment efficacy decreased to 94.4 %. Three and five days after re-treatment efficacies were high with 100 %. At the end of the second treatment week flea activity achieved 92.1%.

Overall, the combination of 4 mg/kg bw Compound 22c (split into 2 x 2 mg/kg bw on two consecutive days)

with 15 mg/kg bw and 10 mg/kg bw Nitenpyram showed an excellent efficacy against fleas. The lower dosage of 5 mg/kg bw Nitenpyram demonstrated a very good efficacy up to 5 days after treatment. 5 mg/kg bw Nitenpyram in combination with Compound 22c showed a good efficacy against fleas.

## Claims

1. Composition for use in the treatment of flea infestations of animals and diseases caused by such infestation comprising an effective amount of an arylpyrazole compound of formula (I)

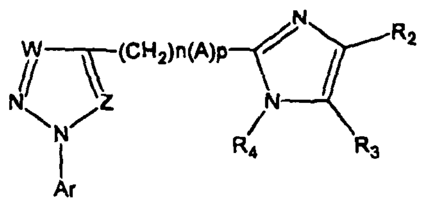

(I)

in which

Ar is 2,6-dichloro-4-trifluoromethylphenyl or 2-nitro-trifluoromethylphenyl;
A is $S(O)_m$, -CH=CH-, O or NH;
W is N and Z is $CR^5$; or W is $CR^1$ and Z is N or $CR^5$;
$R^1$ is hydrogen, optionally substituted alkyl, halogen or $R^{20}S(O)_q$:
$R^2$ and $R^3$ are hydrogen, alkyl, alkenyl or alkynyl, each of which is optionally substituted, aryl, cyano, halogen, nitro, $YR^{20}$, $S(O)_2NR^8R^9$, CHO, $NR^8R^9$ or $CYNR^8R^9$;
$R^4$ is hydrogen, optionally substituted alkyl, optionally substituted alkenyl, acyl or optionally substituted alkoxycarbonyl;
$R^5$ is hydrogen, alkyl, optionally substituted amino or halogen;
$R^8$ and $R^9$ are the same or different and are hydrogen, optionally substituted alkyl, acyl or aryl;
$R^{20}$ is optionally substituted alkyl;
Y is O or S;
m is 0, 1 or 2;
p is 0 or 1;
n is 0, 1 or 2; and
q Is 0, 1 or 2,
and in which a) any alkyl, alkoxy and alkylthio groups are of 1 to 4 carbon atoms; b) any alkenyl or alkynyl groups are of 2 to 5 carbon atoms; c) any substituted alkyl, alkoxy, alkylthio, alkenyl or alkynyl group is substituted by one or more of the same or different groups selected from halogen, $YR^{20}$, dihalocyclopropyl, cyano, nitro, optionally substituted amino, acyloxy and aryl; d) any aryl group is phenyl, optionally substituted, by halogen, alkyl, haloalkyl, alkoxy, haloalkoxy alkylthio, haloalkylthio, haloalkylsulphonyl, cyano or nitro; e) any acyl group Is alkanoyl of 1 to 4 carbon atoms, or alkylsulphonyl or haloalkylsulphonyl; and f) any optionally substituted amino groups are of formula $NR^8R^9$, with the proviso that when W is $CR^1$ and Z is $CR^5$ and n and p are both 0, $R^4$ is not alkyl;
and an effective amount of a nitroenamine compound of formula (II)

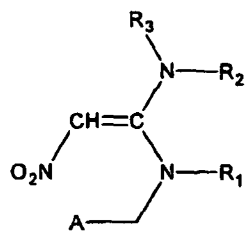

(II)

wherein

R1 is hydrogen, $C_1$-$C_6$ alkyl or $C_3$-$C_7$ cycloalkyl;
R2 is hydrogen, $C_1$-$C_6$ alkyl or $C_3$-$C_7$ cycloalkyl;
R3 is hydrogen or $C_1$-$C_6$ alkyl;
A is heterocyclyl which is unsubstituted or substituted once or repeatedly by Identical or different halogen atoms;
and a physiologically acceptable formulation excipient.

**2.** The composition according to claim 1 **characterized in that** the compound of formula (I) is 5-chloro-1-(2,6-dichloro-4-trifluoromethylphenyl)-4-(4,5-dicyano-1H-imidazol-2-yl)-3-methyl-1-H. pyrazole.

**3.** The composition according to claims 1 or 2 **characterized in that** the compound of formula (II) is N-(6-chloro-pyridin-3ylmethyl)-N-ethyl-N-methyl-2-methyl-2-nitrovinylidenediamine.

**4.** Use of a composition according to claims 1 to 3 as active ingredients for the manufacture of a veterinary medicament for the control of flea infestations on animals.

**5.** Use according to claim 4 **characterized in that** the veterinary medicament comprises the compound of formula (I) and the compound of formula (II) in a single dosage form.

**6.** Use according to claim 4 **characterized in that** the veterinary medicament consists of separate dosage forms of the compounds of formula (I) and of the formula (II) for administration in parallel.

**7.** Use according to claims 4 to 6 **characterized in that** the veterinary medicament is administered to an animal systemically.

**8.** Use according to claims 4 to 7 **characterized in that** the veterinary medicament is in an oral administration form.

**9.** Use according to claims 4 to 8 **characterized in that** the compound of formula (I) is administered in a dose from 0.5 mg /kg bodyweight to 10 mg I kg bodyweight and that of the formula (II) a dose from 1 mg I kg bodyweight to 30 mg I kg bodyweight.

**10.** A kit useful in the treatment of a flea and tick infestation of an animal, which comprises a compound of formula (I) and a compound of formula (II) in a veterinary acceptable formulation and instructions for the control of flea and tick infestations on animals.

## Patentansprüche

**1.** Zusammensetzung zur Verwendung bei der Behandlung von Flohbefall von Tieren und von durch solch einen Befall verursachten Krankheiten, umfassend eine wirksame Menge einer Arylpyrazolverbindung der Formel (I),

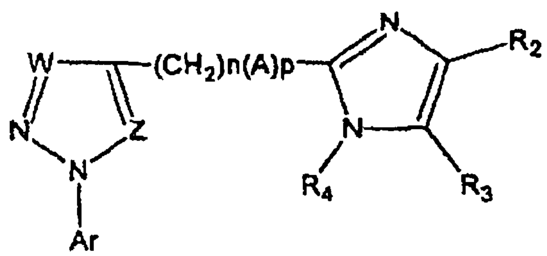

(I)

in der

Ar 2,6-Dichlor-4-trifluormethylphenyl oder 2- Nitro-4-trifluormethylphenyl bedeutet;
A $S(O)_m$, -CH=CH-, O oder NH bedeutet;
W N bedeutet und Z $CR^5$ bedeutet oder W $CR^1$ bedeutet und Z N oder $CR^5$ bedeutet;
$R^1$ Wasserstoff, gegebenenfalls substituiertes Alkyl, Halogen oder $R^{20}S(O)_q$ bedeutet;
$R^2$ und $R^3$ Wasserstoff, Alkyl, Alkenyl oder Alkinyl, die jeweils gegebenenfalls substituiert sind, Aryl, Cyano, Halogen, Nitro, $YR^{20}$, $S(O)_2NR^8R^9$, CHO, $NR^8R^9$ oder $CYNR^8R^9$ bedeuten;

$R^4$ Wasserstoff, gegebenenfalls substituiertes Alkyl, gegebenenfalls substituiertes Alkenyl, Acyl oder gegebenenfalls substituiertes Alkoxycarbonyl bedeutet;
$R^5$ Wasserstoff, Alkyl, gegebenenfalls substituiertes Amino oder Halogen bedeutet;
$R^8$ und $R^9$ gleich oder verschieden sind und Wasserstoff, gegebenenfalls substituiertes Alkyl, Acyl oder Aryl bedeuten,
$R^{20}$ gegebenenfalls substituiertes Alkyl bedeutet;
Y O oder S bedeutet;
m 0, 1 oder 2 bedeutet;
p 0 oder 1 bedeutet;
n 0, 1 oder 2 bedeutet; und
q 0, 1 oder 2 bedeutet,

und in der a) alle Alkyl-, Alkoxy- und Alkylthiogruppen 1 bis 4 Kohlenstoffatome aufweisen; b) alle Alkenyl- oder Alkinylgruppen 2 bis 5 Kohlenstoffatome aufweisen; c) alle substituierten Alkyl-, Alkoxy-, Alkylthio-, Alkenyl- oder Alkinylgruppen durch eine oder mehrere gleiche oder verschiedene Gruppen, ausgewählt aus der Reihe Halogen, $YR^{20}$, Dihalogencyclopropyl, Cyano, Nitro, gegebenenfalls substituiertes Amino, Acyloxy und Aryl substituiert sind; d) alle Arylgruppen Phenyl, gegebenenfalls substituiert durch Halogen, Alkyl, Halogenalkyl, Alkoxy, Halogenalkoxy, Alkylthio, Halogenalkylthio, Halogenalkylsulfonyl, Cyano oder Nitro bedeuten; e) alle Acylgruppen Alkanoyl mit 1 bis 4 Kohlenstoffatomen oder Alkylsulfonyl oder Halogenalkylsulfonyl bedeuten; und f) alle gegebenenfalls substituierten Aminogruppen die Formel $NR^8R^9$ aufweisen, mit der Maßgabe, dass, wenn W $CR^1$ bedeutet und Z $CR^5$ bedeutet und n und p beide 0 bedeuten, $R^4$ nicht Alkyl bedeutet;
und eine wirksame Menge einer Nitroenaminverbindung der Formel (II),

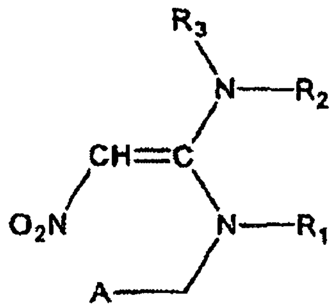

(II)

in der

R1 Wasserstoff, $C_1$-$C_6$-Alkyl oder $C_3$-$C_7$-Cycloalkyl bedeutet;
R2 Wasserstoff, $C_1$-$C_6$-Alkyl oder $C_3$-$C_7$-Cycloalkyl bedeutet;
R3 Wasserstoff oder $C_1$-$C_6$-Alkyl bedeutet;
A Heterocyclyl, das unsubstituiert oder monosubstituiert oder mehrfach durch gleiche oder verschiedene Halogenatome substituiert ist;

und einen physiologisch unbedenklichen Formulierungsgrundstoff.

2. Zusammensetzung nach Anspruch 1, **dadurch gekennzeichnet, dass** es sich bei der Verbindung der Formel (I) um 5-Chlor-1-(2,6-dichlor-4-trifluormethylphenyl)-4-(4,5-dicyano-1H-imidazol-2-yl)-3-methyl-1H-pyrazol handelt.

3. Zusammensetzung nach den Ansprüchen 1 oder 2, **dadurch gekennzeichnet, dass** es sich bei der Verbindung der Formel (II) um N-(6-Chlorpyridin-3-ylmethyl)-N-ethyl-N-methyl-2-methyl-2-nitrovinylidendiamin handelt.

4. Verwendung einer Zusammensetzung nach den Ansprüchen 1 bis 3 als Wirkstoffe für die Herstellung eines tierärztlichen Arzneimittels für die Bekämpfung von Flohbefall an Tieren.

5. Verwendung nach Anspruch 4, **dadurch gekennzeichnet, dass** das tierärztliche Arzneimittel die Verbindung der Formel (I) und die Verbindung der Formel (II) in Einzeldosisform umfasst.

6. Verwendung nach Anspruch 4, **dadurch gekennzeichnet, dass** das tierärztliche Arzneimittel aus getrennten Dosisformen der Verbindungen der Formel (I) und der Formel (II) für die Parallelverabreichung besteht.

7. Verwendung nach den Ansprüchen 4 bis 6, **dadurch gekennzeichnet, dass** das tierärztliche Arzneimittel einem Tier systemisch verabreicht wird.

8. Verwendung nach den Ansprüchen 4 bis 7, **dadurch gekennzeichnet, dass** das tierärztliche Arzneimittel in einer oralen Verabreichungsform vorliegt.

9. Verwendung nach den Ansprüchen 4 bis 8, **dadurch gekennzeichnet, dass** die Verbindung der Formel (I) in einer Dosis von 0,5 mg/kg Körpergewicht bis 10 mg/kg Körpergewicht und die der Formel (II) in einer Dosis von 1 mg/kg Körpergewicht bis 30 mg/kg Körpergewicht verabreicht wird.

10. Kit, das sich für die Behandlung eines Floh- und Zeckenbefalls eines Tiers eignet, und das eine Verbindung der Formel (I) und eine Verbindung der Formel (II) in einer tierärztlich unbedenklichen Formulierung sowie Anweisungen für die Bekämpfung von Floh- und Zeckenbefall an Tieren umfasst.

## Revendications

1. Composition destinée à être utilisée dans le traitement des infestations d'animaux par des puces et des maladies causées par une telle infestation, comprenant une quantité efficace d'un composé arylpyrazole de formule (I)

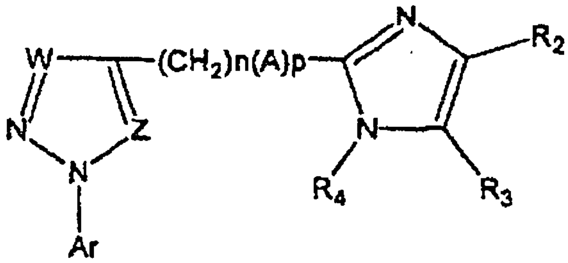

(I)

dans laquelle
Ar est 2,6-dichloro-4-trifluorométhylphényle ou 2-nitro-4-trifluorométhylphényle ;
A est $S(O)_m$, -CH=CH-, O ou NH ;
W est N et Z est $CR^5$ ; ou W est $CR^1$ et Z est N ou $CR^5$;
$R^1$ est hydrogène, alkyle éventuellement substitué, halogène ou $R^{20}S(O)_q$ ;
$R^2$ et $R^3$ sont hydrogène, alkyle, alcényle ou alcynyle, dont chacun est éventuellement substitué, aryle, cyano, halogène, nitro, $YR^{20}$, $S(O)_2NR^8R^9$, CHO, $NR^8R^9$ ou $CYNR^8R^9$;
$R^4$ est hydrogène, alkyle éventuellement substitué, alcényle éventuellement substitué, acyle ou alcoxycarbonyle éventuellement substitué ;
$R^5$ est hydrogène, alkyle, amino éventuellement substitué ou halogène ;
$R^8$ et $R^9$ sont identiques ou différents et sont hydrogène, alkyle éventuellement substitué, acyle ou aryle ;
$R^{20}$ est alkyle éventuellement substitué ;
Y est O ou S ;
m est 0, 1 ou 2 ;
p est 0 ou 1 ;
n est 0, 1 ou 2 ; et
q est 0, 1 ou 2,
et dans laquelle a) tous les groupements alkyle, alcoxy et alkylthio ont de 1 à 4 atomes de carbone ; b) tous les groupements alcényle ou alcynyle ont de 2 à 5 atomes de carbone ; c) tout groupement alkyle, alcoxy, alkylthio, alcényle ou alcynyle est substitué par un ou plusieurs groupements identiques ou différents choisis parmi halogène, $YR^{20}$, dihalogénocyclopropyle, cyano, nitro, amino éventuellement substitué, acyloxy et aryle ; d) tout groupement aryle est phényle, éventuellement substitué, par halogène, alkyle, halogénoalkyle, alcoxy, halogénoalcoxy, alkylthio, halogénoalkylthio, halogénoalkylsulfonyle, cyano
ou nitro ; e) tout groupement acyle est alcanoyle de 1 à 4 atomes de carbone, ou alkylsulfonyle ou halogénoalkylsulfonyle ; et f) tous les groupements amino éventuellement substitués sont de formule $NR^8R^9$, à

condition que lorsque W est $CR^1$ et Z est $CR^5$ et n et p sont tous deux 0, $R^4$ ne soit pas alkyle ;
et une quantité efficace d'un composé nitroénamine de formule (II)

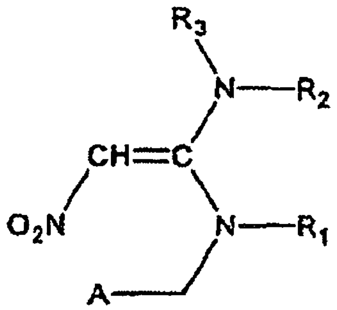

(II)

dans laquelle
R1 est hydrogène, alkyle en $C_1$-$C_6$ ou cycloalkyle en $C_3$-$C_7$ ;
R2 est hydrogène, alkyle en $C_1$-$C_6$ ou cycloalkyle en $C_3$-$C_7$ ;
R3 est hydrogène ou alkyle en $C_1$-$C_6$ ;
A est hétérocyclyle qui est non substitué ou substitué une fois ou de façon répétée par des atomes d'halogène identiques ou différents ;
et un excipient de formulation physiologiquement acceptable.

2. Composition selon la revendication 1, **caractérisée en ce que** le composé de formule (I) est le 5-chloro-1-(2,6-dichloro-4-trifluorométhylphényl)-4-(4,5-dicyano-1H-imidazol-2-yl)-3-méthyl-1-H-pyrazole.

3. Composition selon les revendications 1 ou 2, **caractérisée en ce que** le composé de formule (II) est la N-(6-chloropyridin-3ylméthyl)-N-éthyl-N-méthyl-2-méthyl-2-nitrovinylidènediamine.

4. Utilisation d'une composition selon les revendications 1 à 3, comme principes actifs, pour la fabrication d'un médicament vétérinaire destiné à lutter contre les infestations d'animaux par des puces.

5. Utilisation selon la revendication 4, **caractérisée en ce que** le médicament vétérinaire comprend le composé de formule (I) et le composé de formule (II) sous forme de dosage unitaire.

6. Utilisation selon la revendication 4, **caractérisée en ce que** le médicament vétérinaire est constitué de formes de dosage distinctes des composés de formule (I) et de formule (II) pour l'administration en parallèle.

7. Utilisation selon les revendications 4 à 6, **caractérisée en ce que** le médicament vétérinaire est administré à un animal par voie systémique.

8. Utilisation selon les revendications 4 à 7, **caractérisée en ce que** le médicament vétérinaire est sous une forme d'administration orale.

9. Utilisation selon les revendications 4 à 8, **caractérisée en ce que** le composé de formule (I) est administré à une dose de 0,5 mg/kg de poids corporel à 10 mg/kg de poids corporel et celui de formule (II) une dose de 1 mg/kg de poids corporel à 30 mg/kg de poids corporel.

10. Kit utile dans le traitement d'une infestation d'un animal par des puces et des tiques, **caractérisé en ce qu'**il comprend un composé de formule (I) et un composé de formule (II) dans une formulation acceptable du point de vue vétérinaire et des instructions pour lutter contre les infestations d'animaux par des puces et des tiques.

**Figure 1: Efficacy Ticks**

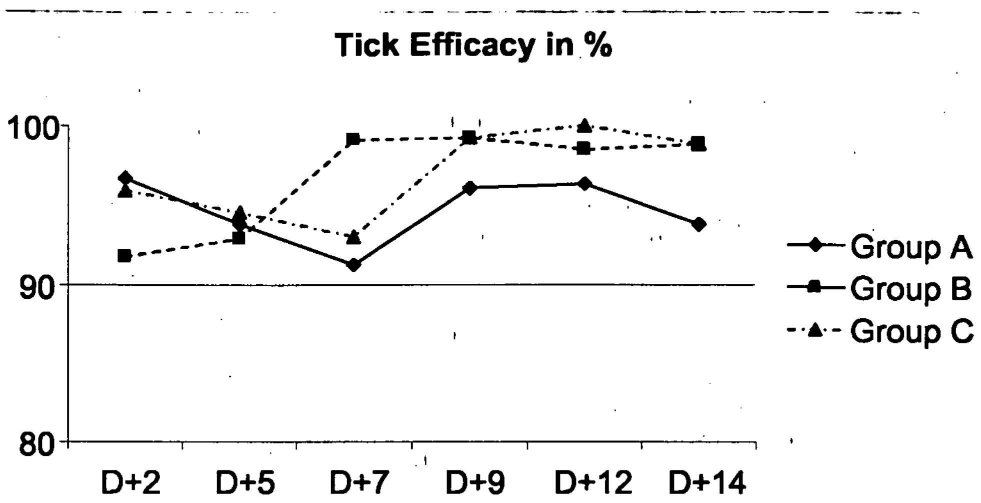


**Figure 2: Efficacy Fleas**

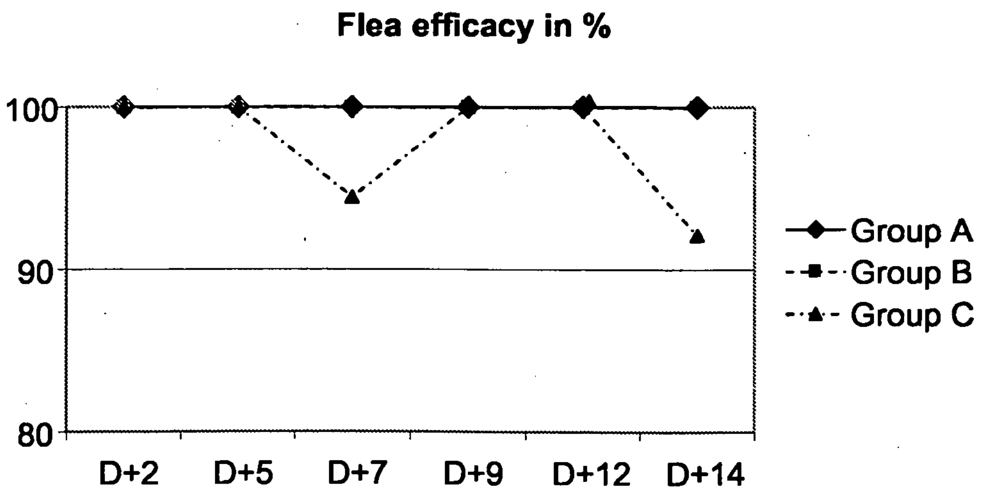

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0412849 A
- EP 0302389 A
- EP 0616494 A
- US 200258683 B


### Non-patent literature cited in the description


- **Dryden MW et al.** *Proceedings of the Annual Meeting of the American Association of Veterinary Parasitology,* 1999, vol. 44, 1-9
- **Rust MK et al.** *J. Med. Entomol.,* 2003, vol. 40 (5), 678-681
- **Gennaro.** Remington: The Science and Practice of Pharmacy. 2000